# EUROPEAN PATENT APPLICATION

(11) **EP 2 875 815 A1**
(43) Date of publication of application: **27.05.2015**
(21) Application number: 13193784.9
(22) Date of filing: 21.11.2013
(51) Int. Cl.: A61K 31/425, A61P 31/00, A61P 31/04

(54) **N'-(2-(3-oxobenzo[d]isothiazol-2(3h)-yl)acetyl)adamantane-1-carbohydrazide as a novel inhibitor for staphylococcus aureus sortase a**

(71) Applicant: Latvian Biomedical Research and Study Centre, 1067 Riga (LV)
(72) Inventor: Zhulenkovs, Dmitrijs, LV-1067 Riga (LV); Rudevica, Zhanna, LV-1067 Riga (LV); Leonciks, Ainars, LV-1067 Riga (LV); Jaudzems, Kristaps, LV-1006 Riga (LV); Zicane, Daina, LV-1007 Riga (LV); Turks, Maris, LV-1007 Riga (LV)
(74) Representative: Kuzjukevica, Lucija

(57) **Abstract**

The present invention relates to a novel sortase A (SrtA) inhibitory compound N'-(2-(3-oxobenzo[d]isothiazol-2(3H)-yl)acetyl)adamantane-1-carbohydrazide which can be used in medicine as anti-infective agent for treating Gram-positive bacterial infection.

## Description

### Subject of the invention

The present invention relates to a novel and selective *S. aureus* sortase A inhibitor, more particularly to a novel sortase A (SrtA) inhibitory compound N'-(2-(3-oxobenzo[d]isothiazol-2(3H)-yl)acetyl)adamantane-1-carbohydrazide which can be used in medicine as anti-infective agent for treating gram-positive bacterial infections.

### Background Art

Gram-positive bacteria are responsible for a large proportion of serious infections worldwide and are among the top ten most frequently isolated organisms associated with healthcare-associated infections [1].

These Gram-positive organisms are found to be among the most commonly isolated organisms in hospitals in Europe, North America, Oceania and Africa [2].

Infections caused by multidrug-resistant Gram-positive bacteria represent a major public health problem worldwide and are consistently associated with high mortality rates. *Staphylococcus spp.* and *Enterococcus* spp. are two of the most common organisms causing nosocomial (hospital-associated) infections today and their resistance to first line therapies such as methicillin and vancomycin continue to rise. Bacterial resistance to existing antibiotics prompts new health threats and it has a significant impact on overall healthcare costs [3], [4].

Nosocomial and community-acquired Methicillin-Resistant *S. aureus* (MRSA) strains and several other staphylococcal species have emerged as a very serious problem in medicine since the 1990s. This group of microorganisms has acquired resistance to methicillin and essentially all other beta-lactam antibiotics [5], [6], [7].

The sortase enzymes are a family of transpeptidases responsible for anchoring the majority of surface proteins to the peptidoglycan cell wall in Gram-positive bacteria [8]. These enzymes are important virulence factors because the surface proteins they display are responsible for bacterial adhesion to host cells and tissues, invasion of host cells, survival, and evasion of immune responses [9], [10].

Two sortase isoforms have been identified in *Staphylococcus aureus,* sortase A (SrtA) and sortase B (SrtB). The SrtB isoform is important in heme iron acquisition and iron homeostasis, whereas the SrtA isoform is essential for the pathogenesis of *S. aureus* and other Gram-positive bacteria [11], [12].

SrtA is a thiol transpeptidase that catalyzes a cell wall sorting reaction in which a surface protein with sorting signal containing a conserved pentapeptide LPXTG motif (Leucine-Proline-any amino acid-Threonine-Glycine) is cleaved between the Threonine and Glycine residues forming an acyl-enzyme intermediate. The resulting threonine carboxyl end of the surface protein is covalently linked to a pentaglycine cross-bridge of peptidoglycan, incorporated into the envelope and displayed on the microbial surface [13], [14], [15] and [16].

*S. aureus* strains lacking the srtA gene (srtA⁻) are unable to retain and display LPXTG proteins at the bacterial cell surface. As a consequence, srtA mutants are significantly less virulent and defective in the establishment of acute infection.

To date there are around 20 known staphylococcal proteins that carry a C-terminal LPXTG sorting signal. These include several important virulence factors that are involved in pathogenicity, such as protein A (Spa), fibronectin-binding proteins A (FnbpA) and B (FnbpB), clumping factors A (ClfA) and B (ClfB), serine-aspartic acid repeat proteins (SdrC, SdrD, SdrE), plasmin-sensitive protein (Pls), and staphylococcal surface proteins (Sas).

Sortase A is not indispensable for microbial growth and viability in cell culture, so the enzyme inhibitors that act as anti-infective agents have limited selective pressure towards the development of bacterial drug resistance and emergence of new antibiotic-resistant strains.

Sortase thus appears to be a very promising target for identifying inhibitors that could be of general use in therapeutics against Gram-positive bacteria [17], [18] and [19].

Screening of small molecule libraries is widely recognized as an effective method for discovering new SrtA inhibitors as drug candidates for the treatment of Gram-positive infections [20]. Some of the newly discovered compounds have demonstrated a significant inhibitory activity in the submicromolar range *in vitro* [21].

### Summary of the invention

The present invention discloses a novel chemical compound with a high inhibitory potential against the gram-positive *S. aureus* sortase A enzyme. The presented chemical compound is new and it effectively inhibits proteolytic cleavage between the Glycine (Gly) and Threonine (Thr) residues of the Leu-Pro-Glu-Thr-Gly, which is a specific motif sequence in the target proteins.

The present invention is a compound of formula (I) 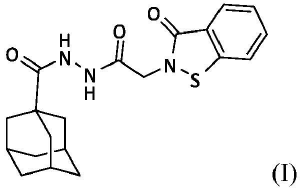 for inhibiting the transpeptidase sortase A (SrtA) from *Staphylococcus aureus* or other Gram-positive bacteria.

Said compound is for use as a medicament, therapeutic agent or potent anti-infective agent in the treatment of bacterial infections.

### Brief description of the drawings

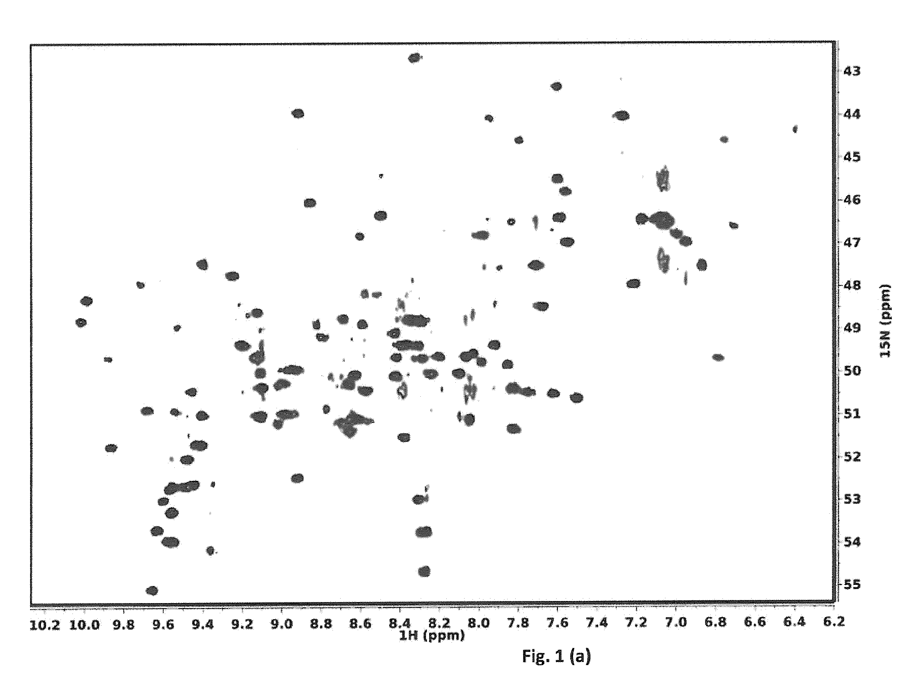

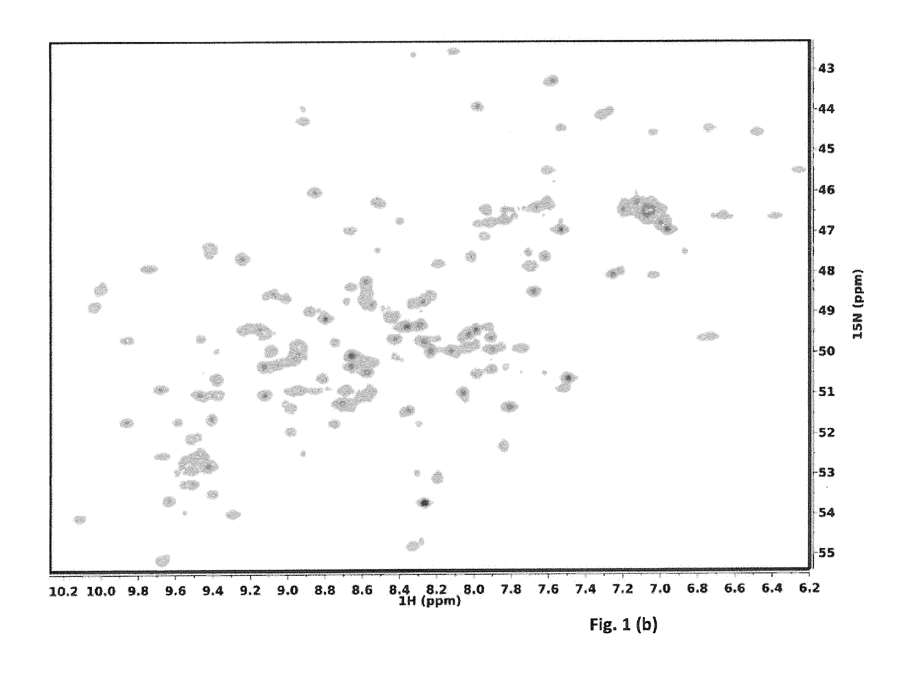

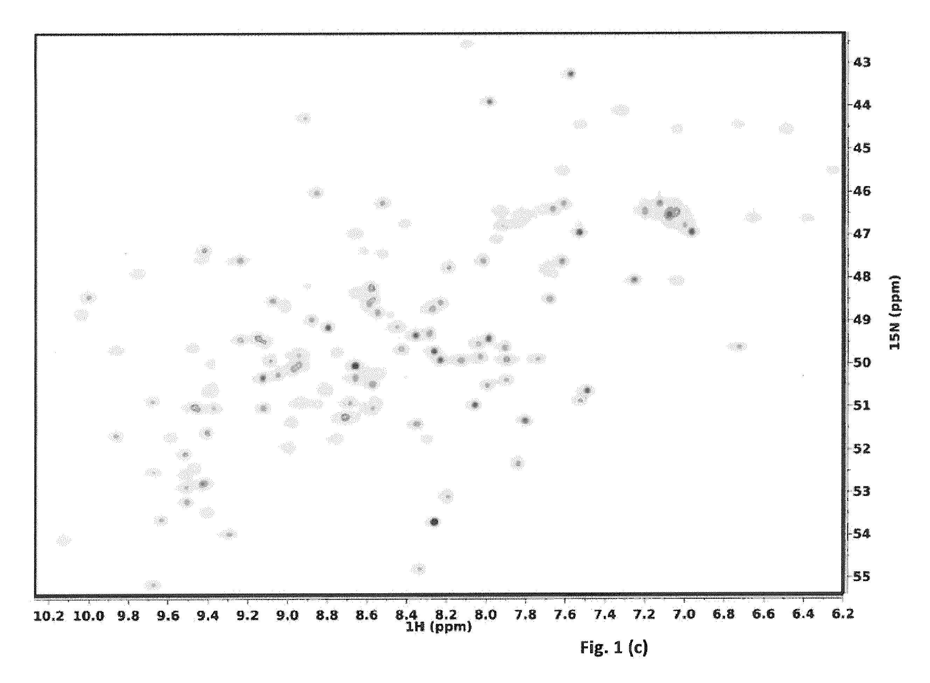

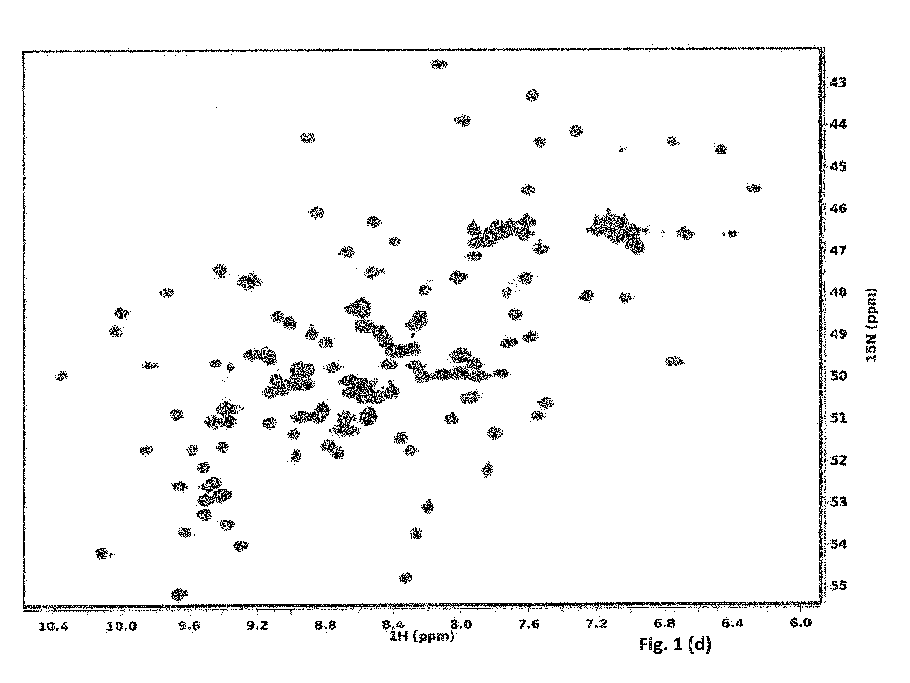

Fig. 1 - represents the binding of a selected inhibitor N-(adamantan-1-yl)-2-(3-oxo-2,3-dihydro-1,2-benzothiazol-2-yl)acetamide to SrtA monitored by 2D [¹⁵N,¹H]-HSQC NMR spectroscopy. (a) Spectrum of SrtA in absence of inhibitor. (b) Spectrum of SrtA in presence of 0.1 mM (0.33 equivalents) inhibitor shows the appearance of a second set of peaks corresponding to the inhibitor-bound form. (c) Spectrum of SrtA in presence of 0.3 mM (1.0 equivalents) inhibitor shows only the inhibitor-bound form. (d) Spectrum of the sample from (c) after dialysis against NMR sample buffer shows almost no differences with respect to the spectrum in (c) indicating that inhibitor binds to SrtA irreversibly.

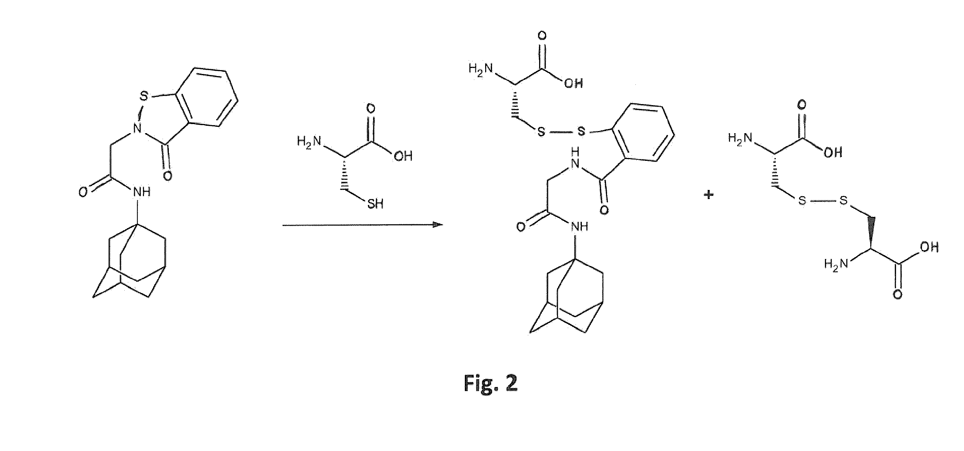

Fig. 2 - represents the observed reaction between inhibitor and cysteine leading to the formation of a covalent adduct.

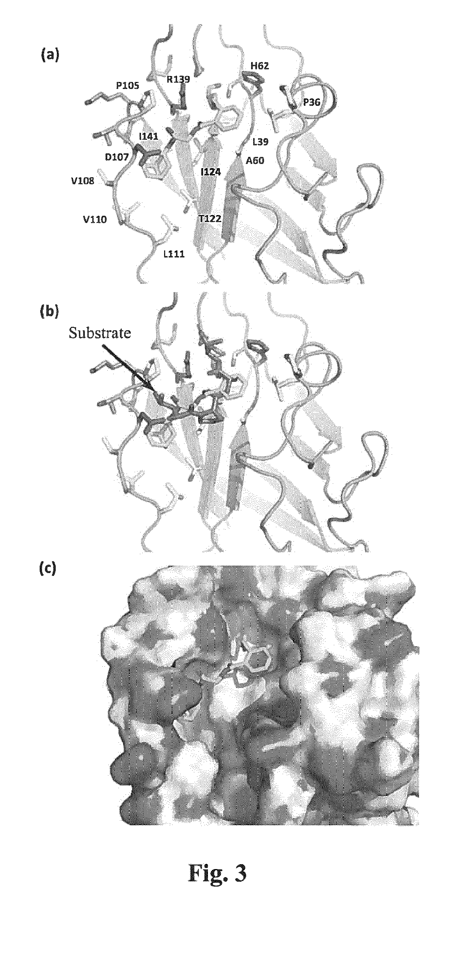

Fig. 3 - NMR structure of the SrtA-inhibitor complex. (a) SrtA active site after modification by inhibitor. The inhibitor (in cyan) and near-by side chains of SrtA are shown with sticks and labeled. Side chains are colored according to their type: hydrophobic in yellow, negatively charged in red, positively charged in blue and hydrophilic in grey. Hydrogens are omitted for clarity. (b) Superposition of the structure of SrtA-inhibitor complex with the structure of substrate analogue LPAT peptide in complex with SrtA shows that inhibitor binds similarly to the substrate analogue. (c) Molecular surface representation of SrtA in complex with inhibitor. It can be seen in the figure that the inhibitor occupies almost all volume of the substrate pocket, the majority of interactions are hydrophobic and hydrogen bond is at the R139.

To identify the new sortase A inhibitor the following steps should be accomplished: high-throughput screening of potent *S. aureus* SrtA inhibitors; determination of inhibitor-enzyme complex structure; synthesis of new derivative of N-(adamantan-1-yl)-2-(3-oxo-2,3-dihydro-1,2-benzothiazol-2-yl)acetamide; and the enzymatic inhibitory activity, minimal bacterial inhibitory concentration and cell cytotoxicity determination of the newly synthesized compound.

The catalytically active recombinant sortase A enzyme used for these studies was expressed with an N-terminal deletion of 59 amino acid residues (SrtA_{ΔNS9}, residues 60-206). The first step included high-throughput screening of potent *S. aereus* SrtA inhibitors and selection of new antibacterial substances as sortase A inhibitors. In order to identify potent inhibitors of *S*. *aureus* sortase A, a high-throughput screening (HTS) of a diverse library of 50,000 drug-like low-molecular weight molecules was performed, which led to the identification of novel promising drug candidates.

After the initial enzymatic control established, the statistical confidence of the analysis method in the high-throughput screening application, based on the variation associated with individual measurements and the dynamic range of the system, was evaluated and the Z' factor was calculated. The Z' factor of the HTS application was equal to 0.82 thus indicating that the system is stable and suitable for screening of sortase inhibitors.

Compounds demonstrating more than 65% SrtA inhibition (45 drug candidates) were identified and subjected to secondary screening under the same conditions to limit variability. One of them, N-(adamantan-1-yl)-2-(3-oxo-2,3-dihydro-1,2-benzothiazol-2-yl)acetamide was validated as a best hit molecule and selected for further investigation. The formula of N-(adamantan-1-yl)-2-(3-oxo-2,3-dihydro-1,2-benzothiazol-2-yl)acetamide (II) is as follows:

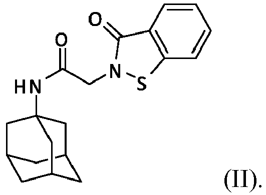

For this inhibitor, the concentration required to reduce the activity of sortase A enzyme by 50% was determined and the IC₅₀ value was equal to 6.2 µM. This molecule was used as the basis to develop further new SrtA inhibitors with increased activity.

The next step included determination of the inhibitor-enzyme complex structure. Further structural studies were performed to elucidate the precise interaction site where the selected inhibitor binds to the recombinant sortase A, give insight into the mode of action and propose possible chemical modifications to increase the inhibitory activity versus the SrtA enzyme.

Determination of the complex structure of selected inhibitor and sortase enzyme was performed by using the nuclear magnetic resonance (NMR) spectroscopy methods. The collected NMR data allowed to specify the structure of inhibitory complexes.

The inhibitor molecule demonstrated irreversible binding to the active site of SrtA as manifested by large NMR chemical shift perturbations of the active site residues as well as by virtually no changes in the 2D 15N-1H HSQC spectrum of the SrtA-inhibitor complex after dialysis against NMR buffer (Figure 1). To verify that the selected inhibitor can react with a cysteine thiol (such as the SrtA active site C 184) we performed a reaction with cysteine in the NMR tube and observed the formation of a covalent adduct (Figure 2). The chemical structure of the product was verified using proton 1D and 2D NMR methods.

In order to gain insights into the mechanism of inhibition, the three-dimensional structure of SrtA bound with inhibitor by NMR spectroscopy was determined. The structure showed that inhibitor is covalently linked to SrtA via a disulfide bridge formed between the SrtA active site cysteine (C184) thiol and the inhibitor's benzo[d]isothiazol-3-one moiety's sulfur atom, thereby resulting in the cleavage of its nitrogen-sulfur bond (Figure 3).

Furthermore, it was established that the resulting 2-mercapto-benzamide moiety's carbonyl group of the inhibitor forms a hydrogen bond with R197 and the phenyl group interacts with residues L97 and A118, while the adamantyl moiety is buried in a hydrophobic cleft, where it interacts with residues V166, V168, L169 of the β6/β7 loop as well as I182 and I199. The β6/β7 loop of SrtA undergoes significant conformational changes upon reaction with inhibitor, suggesting an induced fit mechanism.

Based on the three-dimensional structure of the SrtA-inhibitor complex, several chemical modifications to the molecular structure of the inhibitor were realized.

A substituent was inserted in the benzo[d]isothiazol-3-one moiety at positions 4, 5, and 6 since this part of the inhibitor does not form tight contacts with the protein. It was suggested that the additional interactions might improve inhibition and selectivity.

The length of the linker between the benzo[d]isothiazol-3-one and adamantyl moieties was varied in order to increase the strength of interaction between the adamantyl moiety with hydrophobic residues in the cleft region of SrtA (V166, V168, L169, I182 and I199). The structure suggested an induced fit mechanism and therefore the optimal length of the linker might be determined experimentally. Additionally, a negatively charged group was inserted in the linker to form a salt bridge with R197.

The method for synthesis of new derivative of N-(adamantan-1-yl)-2-(3-oxo-2,3-dihydro-1,2-benzothiazol-2-yl)acetamide included the following steps: expression of the recombinant sortase A; FRET-based high-throughput screening of chemical library; determination of inhibitor-enzyme complex structure and new inhibitor design; synthesis of the new inhibitor with increased inhibitory activity; and testing of inhibitory properties of the newly synthesized compound.

A new synthesis of derivatives of the best selected compound was performed. A resulting new substance with improved characteristics was synthesized at milimolar amounts for further studies to determine its toxicity and efficiency.

Adamantane derivative *N*-(2-(3-oxobenzo[d]isothiazol-2(3H)-yl)acetyl)adamantane-1-carbohydrazide **(6)** was synthesized according to the following scheme:

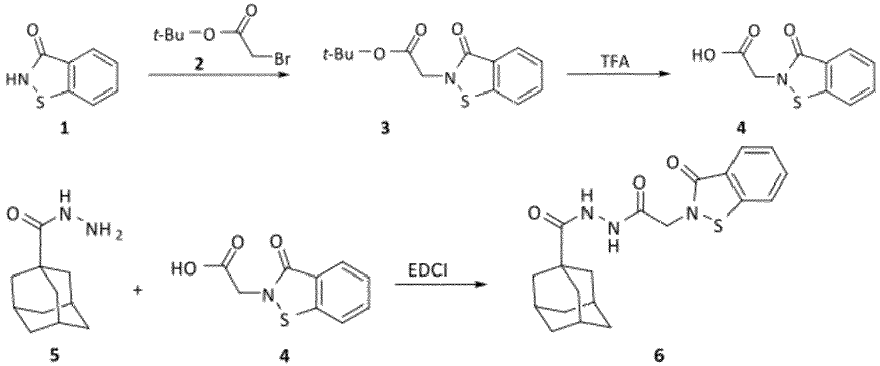

The corresponding acetic acid derivative **(4)** was obtained in two steps according to the known procedure.

Benzo[d]isothiazol-3(2*H*)-one **(1)** was transformed into *tert*-butyl 2-(3-oxobenzo[d]isothiazol-2(3*H*)-yl)acetate **(3)** by treatment with *tert*-butyl 2-bromoacetate **(2).** The latter was deprotected in acidic conditions (trifluoroacetic acid) and yielded expected 2-(3-oxobenzo[d]isothiazol-2(3*H*)-yl)acetic acid **(4).**

2-(3-Oxobenzo[d]isothiazol-2(3*H*)-yl)acetic acid **(4)** was further condensed with adamantane-1-carbohydrazide **(5)** to yield the aforementioned final product.

*N*-Ethyl-*N'*-(3-dimethylaminopropyl)carbodiimide hydrochloride (EDCI) (0.16 g, 0.83 mmol, 1.15 equiv.) and adamantane-1-carbohydrazide **(5)** (0.15 g, 0.77 mmol, 1.07 equiv.) were successively added to a solution of 2-(3-oxobenzo[d]isothiazol-2(3*H*)-yl)acetic acid **(4)** (0.15 g, 0.72 mmol, 1.00 equiv.) in anhydrous DMF (5 mL) at ambient temperature. The resulting reaction mixture was stirred at ambient temperature for 21 h. The solvent was evaporated under reduced pressure and the residue was partitioned between water (10 mL) and ethyl acetate (20 mL). The aqueous phase was additionally extracted with ethyl acetate (3 × 5 mL). The combined organic layer was successively washed with 5% aqueous solution of citric acid (7 mL), 5% aqueous solution of NaHCO₃ (7 mL), brine (10 mL), dried over anhydrous Na₂SO₄, filtered and evaporated under reduced pressure. Crystallization of the crude product from a mixture consisting from ethyl acetate and hexanes provided 61 mg (22%) of N-(2-(3-oxobenzo[d]isothiazol-2(3*H*)-yl)acetyl)adamantane-1-carbohydrazide **(6).**

The H-NMR (DMSO-*d6*, 300 MHz) is: 10.03 (s, 1H, NH), 9.42 (s, 1H, NH), 7.98 (d, 1H, ³*J* = 7.8 Hz, H-C(Ar)), 7.88 (d, 1H, ³*J* = 7.8 Hz, H-C(Ar)), 7.70 (t, 1H, ³*J* = 7.8 Hz, H-C(Ar)), 7.44 (t, 1H, ³*J*=7.8 Hz, H-C(Ar)), 4.53 (s, 2H, CO-CH₂-N), 2.02-1.92 (m, 3H, H-C(adam.)), 1.89-1.85 (m, 1H, H-C(adam.)), 1.83-1.78 (m, 5H, H-C(adam.)), 1.72-1.60 (m, 6H, H-C(adam.)).

The newly synthesized compound N'-(2-(3-oxobenzo[d]isothiazol-2(3H)-yl)acetyl)adamantane-1-carbohydrazid was tested in three different assays to evaluate its inhibitory potential in comparison to the initially selected hit molecule, N-(adamantan-1-yl)-2-(3-oxo-2,3-dihydro-1,2-benzothiazol-2-yl)acetamide.

These assays included determination of 50% SrtA inhibition concentration (IC₅₀) of the newly synthesized derivative in dose-response experiments using the recombinant sortase A enzyme incubated with FRET peptide substrate containing the LPETG motif, minimal inhibitory concentration (MIC) test with two staphylococcal species to measure the antimicrobial efficacy of the new inhibitory molecule, and toxicity test using a normal mouse embryonic fibroblasts cell line NIH/3T3 to determine the cell growth IC₅₀ inhibitory concentration.

The minimum inhibitory concentration was defined as the concentration of an antimicrobial agent that completely inhibited cell growth during a 24-hour incubation at 37°C. The MIC values of test compounds that inhibited cell growth of Gram-positive *Staphylococcus aureus* and *S. epidermidis* were determined by broth dilution micromethod. A series of solutions with a range from 0.1 µg/ml to 400 µg/ml were made in using a logarithmical phase culture with OD₆₀₀ = 0.1 (corresponds to ∼1.2 x 10⁸ CFU/ml in 96-well plate standard. The plate was incubated at 37 °C for 24 hours and growth was assayed with a microplate reader by monitoring the absorption at 600 nm. All experiments were done in triplicate, and repeated three times.

The growth inhibitory effect of the newly synthesized compound was studied on NIH/3T3mouse fibroblast cell line. NIH/3T3 cells were seeded into 96-well microplates with initial density of 1x10⁴ cells per well in DMEM culture medium with 10% FCS. After 24 hours of incubation, cells were exposed to a graded series of concentrations of chemical compounds for additional 48 hours. The cell medium was removed and 100 µl of fresh culture medium without phenol red and 1 mM MTT solution was added to each well. After 3 hours of incubation, an equal volume of 1% SDS-HCl solution was added and plates were incubated for additional 4 hours. Absorbance was measured at 570 nm and the data were fitted to a four parameter variable slope equation to determine the IC₅₀ value.

The new compound exhibited improved inhibitory activity in comparison to the initial hit molecule, 3.8 µM against 6.1 µM, in SrtA enzyme FRET inhibition assay. The results of are presented in Table 1. Values are means ±SD (n = 5).

**TABLE 1**

| Inhibitory effect of selected compounds on the activity of SrtA | |
|---|---|
| **Compound** | **SrtA inhib. concentration, IC₅₀ (µM)** |
| N-(adamantan-1-yl)-2-(3-oxo-2,3-dihydro-1,2-benzothiazol-2-yl)acetamide | 6.2 ± 0.64 |
| N'-(2-(3-oxobenzo[d]isothiazol-2(3H)-yl)acetyl)adamantane-1-carbohydrazide | 3.8 ± 0.10 |

The antibacterial MIC values were measured by the broth microdilution method and are presented in Table 2.

The newly synthesized compound and the initial hit molecule demonstrated significant inhibitory activity against two Gram-positive pathogenic strains that are associated with nosocomial infections, *Staphylococcus aureus* and *Staphylococcus epidermidis.* The newly synthesized compound, N'-(2-(3-oxobenzo[d]isothiazol-2(3H)-yl)acetyl)adamantane-1-carbohydrazide, exhibited increased inhibitory activity against *S. epidermidis* in comparison to *S. aureus* strain, 4 µg/ml and 16 µg/ml, respectively. The MIC values of the new compound were an average 10 fold lower in comparison to the initial hit molecule, N-(adamantan-1-yl)-2-(3-oxo-2,3-dihydro-1,2-benzothiazol-2-yl)acetamide (16 and 1 µg/ml against *S. aureus* and 4 and 0.5 µg/ml against and *S. epidermides,* respectively).

**TABLE 2**

| Minimal inhibitory activity of two SrtA inhibitors against two Staphylococcus strains | | |
|---|---|---|
| **Compound** | **MIC, S.aureus (µg/ml)** | **MIC, S. epidermidis (µg/ml)** |
| N-(adamantan-1-yl)-2-(3-oxo-2,3-dihydro-1,2-benzothiazol-2-yl)acetamide | 1 | 0.5 |
| N'-(2-(3-oxobenzo[d]isothiazol-2(3H)-yl)acetyl)adamantane-1-carbohydrazide | 16 | 4 |

The last objective was to evaluate the cytotoxic effect of the new inhibitory N'-(2-(3-oxobenzo[d]isothiazol-2(3H)-yl)acetyl)adamantane-1-carbohydrazide. The cytotoxic response is dependent on the cell line tested and NIH/3T3 mouse fibroblasts are a well-characterized cellular model used to study toxicity and drug delivery processes. The results are summarized in Table 3. In the table 3 data are given as the mean of three independent experiments ±SD.

The newly synthesized compound exhibited a cytotoxic activity with IC₅₀ value of 12.1 µg/ml against NIH/3T3 cells that was significantly lower than for the initially selected N-(adamantan-1-yl)-2-(3-oxo-2,3-dihydro-1,2-benzothiazol-2-yl)acetamide hit compound (1.3 µg/ml; Table 3).

**TABLE 3**

| Cytotoxic activity of two SrtA inhibitors | |
|---|---|
| **Compound** | **50% NIH/3T3 cell growth inhibition concentration, (IC₅₀, µg/ml)** |
| N-(adamantan-1-yl)-2-(3-oxo-2,3-dihydro-1,2-benzothiazol-2-yl)acetamide | 1.3 ± 0.2 |
| N'-(2-(3-oxobenzo[d]isothiazol-2(3H)-yl)acetyl)adamantane-1-carbohydrazide | 12.1 ± 1.5 |

### References cited in the description

1. Sievert DM, Ricks P, Edwards JR, Schneider A, Patel J, Srinivasan A, Kallen A, Limbago B, Fridkin, S. Antimicrobial-resistant pathogens associated with healthcare-associated infections: summary of data reported to the national healthcare safety network at the centers for disease control and prevention, 2009-2010. Infect Control Hosp Epidemiol. (2013) 34(1):1-14.
2. Vincent JL, Rello J, Marshall J, Silva E, Anzueto A, Martin CD, Moreno R, Lipman J, Gomersall C, Sakr Y, Reinhart K; EPIC II Group of Investigators. International study of the prevalence and outcomes of infection in intensive care units. JAMA. (2009) 302(21):2323-9.
3. Woodford N, Livermore DM.J Infect. Infections caused by Gram-positive bacteria: a review of the global challenge. 2009;59 Suppl 1:S4-16.
4. Rybak JM, Barber KE, Rybak MJ. Current and prospective treatments for multidrug-resistant gram-positive infections. Expert Opin Pharmacother. 2013;14(14):1919-32.
5. Fitzgerald JR, Sturdevant DE, Mackie SM, Gill SR, Musser JM. Evolutionary genomics of Staphylococcus aureus: insights into the origin of methicillin-resistant strains and the toxic shock syndrome epidemic. Proc Natl Acad Sci USA. 2001;98(15):8821-6.
6. Tenover FC, Goering RV. Methicillin-resistant Staphylococcus aureus strain USA300: origin and epidemiology. J Antimicrob Chemother. 2009;64(3):441-6.
7. Otter JA, French GL. Molecular epidemiology of community associated meticillin-resistant Staphylococcus aureus in Europe. Lancet Infect Dis. 2010;10(4):227-39.
8. Comfort D, Clubb RT. A comparative genome analysis identifies distinct sorting pathways in gram-positive bacteria. Infect Immun. 2004;72(5):2710-22.
9. Navarre WW, Schneewind O. Surface proteins of gram-positive bacteria and mechanisms of their targeting to the cell wall envelope. Microbiol Mol Biol Rev. 1999;63(1):174-229.
10. Alksne LE, Projan SJ. Bacterial virulence as a target for antimicrobial chemotherapy. Curr Opin Biotechnol. 2000 Dec;11(6):625-36.
11. Mazmanian SK, Skaar EP, Gaspar AH, Humayun M, Gornicki P, Jelenska J, Joachmiak A, Missiakas DM, Schneewind O. Passage of heme-iron across the envelope of Staphylococcus aureus. Science. 2003;299(5608):906-9.
12. Paterson GK, Mitchell TJ. The biology of Gram-positive sortase enzymes. Trends Microbiol. 2004;12(2):89-95.
13. Schneewind O, Fowler A, Faull KF. Structure of the cell wall anchor of surface proteins in Staphylococcus aureus. Science. 1995;268(5207):103-6.
14. Ton-That H, Liu G, Mazmanian SK, Faull KF, Schneewind O. Purification and characterization of sortase, the transpeptidase that cleaves surface proteins of Staphylococcus aureus at the LPXTG motif. Proc. Natl. Acad. Sci. U.S.A. 1999;96(22):12424-9.
15. Cossart P, Jonquières R. Sortase, a universal target for therapeutic agents against gram-positive bacteria? Proc Natl Acad Sci USA. 2000;97(10):5013-5.
16. Perry AM, Ton-That H, Mazmanian SK, Schneewind O. Anchoring of surface proteins to the cell wall of Staphylococcus aureus. III. Lipid II is an in vivo peptidoglycan substrate for sortase-catalyzed surface protein anchoring. J Biol Chem. (2002) 277(18):16241-8.
17. Mazmanian SK, Liu G, Jensen ER, Lenoy E, Schneewind O. Staphylococcus aureus sortase mutants defective in the display of surface proteins and in the pathogenesis of animal infections. Proc Natl Acad Sci USA. (2000);97(10):5510-5.
18. Weiss WJ, Lenoy E, Murphy T, Tardio L, Burgio P, Projan SJ, Schneewind O, Alksne L. Effect of srtA and srtB gene expression on the virulence of Staphylococcus aureus in animal models of infection. J Antimicrob Chemother. (2004);53(3):480-6.
19. Tsompanidou E, Denham EL, Sibbald MJ, Yang XM, Seinen J, Friedrich AW, Buist G, van Dijl JM. The sortase A substrates FnbpA, FnbpB, ClfA and ClfB antagonize colony spreading of Staphylococcus aureus. PLoS One. 2012;7(9):e44646.
20. Maresso AW, Wu R, Kern JW, Zhang R, Janik D, Missiakas DM, Duban ME, Joachimiak A, Schneewind O. Activation of inhibitors by sortase triggers irreversible modification of the active site. J Biol Chem. 2007;282(32):23129-39.
21. Suree N, Yi SW, Thieu W, Marohn M, Damoiseaux R, Chan A, Jung ME, Clubb RT. Discovery and structure-activity relationship analysis of Staphylococcus aureus sortase A inhibitors. Bioorg Med Chem. 2009;17(20):7174-85.

## Claims

1. A compound of formula (I) 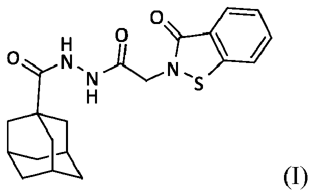 for inhibiting *Staphylococcus aureus* sortase A enzyme.

2. The compound according to Claim 1, wherein said compound is for use as a medicament, therapeutic agent or potent anti-infective agent in the treatment of bacterial infections.
